# EUROPEAN PATENT APPLICATION

(11) **EP 4 545 978 A1**
(43) Date of publication of application: **30.04.2025**
(21) Application number: 23826824.7
(22) Date of filing: 10.05.2023
(51) Int. Cl.: G01N 37/00, G01N 35/08, C12Q 1/68

(54) **SUBSTRATE FOR INSPECTION DEVICE, INSPECTION DEVICE, AND METHOD FOR MANUFACTURING INSPECTION DEVICE**

(30) Priority: 23.06.2022 JP 2022100746
(71) Applicant: Dexerials Corporation, Shimotsuke-shi, Tochigi 323-0194 (JP)
(72) Inventor: MONJU, Takuya, Shimotsuke-shi, Tochigi 323-0194 (JP); HIRAKAWA, Manabu, Shimotsuke-shi, Tochigi 323-0194 (JP)
(74) Representative: Ter Meer Steinmeister & Partner
(86) International application number: PCT/JP2023/017509
(87) International publication number: WO 2023/248635

(57) **Abstract**

There is provided a substrate for an inspection device. The substrate has an average thickness of 250 µm or greater and 350 µm or less, and a density of 0.25 × 10⁶ g/m³ or greater and 0.40 × 10⁶ g/m³ or less.

## Description

### TECHNICAL FIELD

The present invention relates to a substrate for an inspection device, an inspection device, and a method for manufacturing an inspection device.

### BACKGROUND ART

In recent years, measurement methods for POCT (Point of Care Testing; immediate diagnosis at a clinical site) have been rapidly spreading. As a representative example, an inspection device (may be referred to as an "inspection chip," "inspection device," or the like hereinafter) using a measurement method called an immunochromatography method in which the principle of sandwich ELISA and the principle of chromatography are combined has been used.

As such an inspection device, for example, a sheet-like inspection chip has been proposed (see, for example, Patent Document 1). The inspection chip includes a first layer on a front surface side and a second layer on a back surface side, where the first layer and the second layer are adjacent to each other, either the first layer or the second layer includes a fluid-receiving portion, the first layer includes at least a detection confirmation portion, and the second layer includes at least a fluid-flow portion adjacent to the detection confirmation portion, and a fluid flow path communicating with the fluid-flow portion.

In the case where the first layer of the inspection chip of Patent Document 1 includes the fluid-receiving portion, the fluid-receiving portion is set apart from the detection confirmation portion, and when an inspection target fluid is dropped into the fluid-receiving portion, the inspection target fluid flows through the fluid-receiving portion, the fluid flow path, and the fluid-flow portion in this order toward the detection confirmation portion due to capillary action.

### CITATION LIST

### PATENT DOCUMENTS

Patent Document 1: Japanese Unexamined Patent Application Publication No. 2021-175970

### SUMMARY OF THE INVENTION

### TECHNICAL PROBLEM

Since a flow path is formed by heat-transferring a heat transfer medium to a substrate in the inspection chip of Patent Document 1, the inspection chip retains a hydrophobic material (ink) including a hydrophobic substance as the heat transfer medium, and there is a limit in an amount of the ink with which the substrate (e.g., filter paper) is impregnated. Even if an amount of the ink for impregnation is increased for improving the impregnation performance with the ink, the ink also penetrates in the planar direction, which may impair the precision of the flow path. In addition, an average thickness of a substrate in which a flow path can be formed is restricted, and a thinner substrate is preferred for formation of a flow path.

On the other hand, the substrate needs a certain degree of strength (paper strength) in view of handling. In order to impart paper strength, a higher cellulose density is more preferred. However, a substrate that is thin and has a high density reduces flowability of an inspection target, and therefore such a substrate is not suitable for a highly viscous inspection target or a suspended inspection target. In particular, raw milk including large milk fat globules causes a problem such that the milk fat globules remain between fibers constituting the substrate and block the flow path.

The present invention aims to solve the above various problems existing in the related art, and achieve the following object. Specifically, the present invention has an object to provide a substrate for an inspection device, an inspection device using the substrate for the inspection device, and a method for manufacturing an inspection device, which can achieve both formability of a flow path and flowability of a highly viscous inspection target or a suspended inspection target.

### SOLUTION TO THE PROBLEM

Means for solving the above problems are as follows.
<1> A substrate for an inspection device, the substrate having:
   an average thickness of 250 µm or greater and 350 µm or less; and
   a density of 0.25 × 10⁶ g/m³ or greater and 0.40 × 10⁶ g/m³ or less.
<2> The substrate for the inspection device according to <1>,
   wherein the substrate has a basis weight of 50 g/m² or greater and 100 g/m² or less.
<3> The substrate for the inspection device according to <1> or <2>,
   wherein the substrate includes at least one selected from a group consisting of filter paper, woodfree paper, a nonwoven fabric, and nitrocellulose.
<4> An inspection device for inspecting a presence or absence of a target substance in an inspection target, the inspection device including:
   a hydrophobic material; and
   the substrate for the inspection device of <1> or <2>, impregnated with the hydrophobic material,
   wherein the inspection device includes a hydrophobic portion impregnated with the hydrophobic material, and an exposed portion in which the substrate is exposed without being impregnated with the hydrophobic material.
<5> The inspection device according to <4>,
   wherein the exposed portion includes a fluid-receiving portion, a flow path portion communicating with the fluid-receiving portion, and a detection portion communicating with the flow path portion.
<6> The inspection device according to <4> or <5>,
   wherein the hydrophobic portion and the exposed portion are provided to both sides of the substrate for the inspection device, and
   flow paths having mutually different designs are formed in respective sides of the substrate for the inspection device, and the flow paths communicate with each other to form a three-dimensional flow path.
<7> The inspection device according to any one of <4> to <6>,
   wherein an amount of the hydrophobic material with which the substrate for the inspection device is impregnated is 15 g/m² or greater and 20 g/m² or less.
<8> The inspection device according to any one of <4> to <7>,
   wherein the inspection device is for inspecting an emulsion including droplets having a particle size of 1 µm or greater.
<9> The inspection device according to any one of <4> to <8>,
   wherein the inspection device is for inspecting unhomogenized milk.
<10> A method for manufacturing an inspection device, the method including:
   impregnating both sides of the substrate for the inspection device of any one of <1> to <9> with a hydrophobic material to form a hydrophobic portion impregnated with the hydrophobic material and an exposed portion in which the substrate for the inspection device is exposed without being impregnated with the hydrophobic material.

### EFFECTS OF THE INVENTION

According to the present invention, the above various problems existing in the related art can be solved, the above object can be achieved, and a substrate for an inspection device, an inspection device using the substrate for the inspection device, and a method for manufacturing an inspection device, which can achieve both formability of a flow path and flowability of a highly viscous inspection target or a suspended inspection target, can be provided.

### BRIEF DESCRIPTION OF THE DRAWINGS

[Fig. 1] Fig. 1 is a schematic view illustrating one example of an inspection device of the present invention.
[Fig. 2] Fig, 2 is a schematic view illustrating one example of a heat transfer medium.
[Fig. 3A] Fig, 3A is a schematic view illustrating one example of a flow path pattern as viewed from a flow path side.
[Fig. 3B] Fig. 3B is a schematic view illustrating one example of a flow path pattern as viewed from a detection portion side.
[Fig. 4A] Fig. 4A is a view illustrating a state of Example 1 in which leakage of the inspection target to the flow path did not occur, and desired barrier properties were achieved.
[Fig. 4B] Fig. 4B is a view illustrating a state of Comparative Example 2 in which leakage of the inspection target to the flow path occurred, and barrier properties were not desirable.

### DESCRIPTION OF EMBODIMENTS

### (Substrate for inspection device)

The substrate for the inspection device of the present invention is a substrate that is used in an inspection device for inspecting a presence or absence of a target substance in an inspection target. In the present invention, both formability of a flow path, and flowability of a highly viscous inspection target or a suspended inspection target can be achieved by optimizing an average thickness and density of the substrate for the inspection device, and moreover a basis weight of the substrate for the inspection device.

The average thickness of the substrate for the inspection device is 250 µm or greater and 350 µm or less, and preferably 250 µm or greater and 300 µm or less. The average thickness can be measured, for example, according to JIS P8118:2014 "Paper and board - Determination of thickness, density and specific volume."

The density of the substrate for the inspection device is 0.25 × 10⁶ g/m³ or greater and 0.40 × 10⁶ g/m³ or less, and preferably 0.25 × 10⁶ g/m³ or greater and 0.35 × 10⁶ g/m³ or less. The density can be calculated by dividing a basis weight of the substrate for the inspection device by the average thickness of the substrate for the inspection device.

The basis weight of the substrate for the inspection device is preferably 50 g/m² or greater and 100 g/m² or less, and more preferably 75 g/m² or greater and 95 g/m² or less. The basis weight is a value measured according to JIS P8124:2011 "Paper and board - Determination of grammage."

A material of the substrate for the inspection device is not particularly limited, and may be appropriately selected according to the intended purpose. Examples of the material of the substrate include filter paper (e.g., membranes, etc.), plain paper, woodfree paper, watercolor paper, Kent paper, synthetic paper, synthetic resin films, special paper having a coating layer, nitrocellulose, fabrics, textile products, and the like. The above materials may be used alone or in combination of two or more. Among the above materials, filter paper (e.g. membranes, etc.), woodfree paper, and fabrics are preferred.

Examples of the fabrics include artificial fibers (e.g., rayon, Bemberg, acetate, nylon, polyester, vinylon, etc.), natural fibers (e.g., cotton, silk, etc.), mixed fibers of the foregoing, nonwoven fabrics of the foregoing, and the like.

A shape of the substrate for the inspection device is not particularly limited, and may be appropriately selected according to the intended purpose. Examples of the shape of the substrate for the inspection device include sheet shapes, strip shapes, and the like.

A structure of the substrate for the inspection device is not particularly limited, and may be appropriately selected according to the intended purpose.

Since the substrate for the inspection device of the present invention can achieve both formability of a flow path and flowability of a highly viscous inspection target or a suspended inspection target, the substrate for the inspection device of the present invention can be suitably used as a substrate of the inspection device of the present invention as described below.

### (Inspection device)

The inspection device of the present invention is an inspection device that inspects a presence or absence of a target substance in an inspection target. The inspection device of the present invention includes a hydrophobic material and the substrate for the inspection device of the present invention, which is impregnated with the hydrophobic material. The inspection device includes a hydrophobic portion impregnated with the hydrophobic material, and an exposed portion in which the substrate for the inspection device is exposed without being impregnated with the hydrophobic material.

### <Inspection target>

The inspection target is not particularly limited as long as the inspection target is a fluid, and may be appropriately selected according to the intended purpose. Examples of the inspection target include blood (serum and plasma), lymphatic fluids, urine, spinal fluids, nasal fluids, saliva, specimen extract, and the like. As the inspection target, any animal, including humans, is targeted.

### <Target substance>

The target substance is not particularly limited, and may be appropriately selected according to the intended purpose. Examples of the target substance include cells, nucleic acids, proteins, amino acids, carbohydrates, lipids, tumor markers, inflammation markers, enzymes, hormones, signal transducers, and the like.

Examples of the cells include circulating tumor cells (CTCs), erythrocytes, leukocytes, and the like.

Examples of the nucleic acids include circulating normal DNAs, circulating tumor DNAs, non-coding RNAs (miRNA, transfer RNA, ribosomal RNA, etc.), and the like.

Examples of the proteins include albumin, hemoglobin, γ-globulin, fibrinogen, antithrombin III, transferrin, ceruloplasmin, cytokine including a growth factor, chemokine, and the like.

Examples of the carbohydrates include dextrose, 1.5 anhydroglycitol (1.5 AG), and the like.

Examples of the lipids include neutral fat, HDL cholesterol, LDL cholesterol, and the like.

The tumor markers are not particularly limited, and may be appropriately selected according to the intended purpose. Examples of the tumor markers include α-fetoprotein (AFP), carcinoembryonic antigens (CEAs), CA19-9 (sialyl Lewis A sugars), prostate-specific antigens (PSAs), CA125 (glycoprotein), squamous cell carcinoma (SSC) related antigens, and the like.

The inflammation markers are not particularly limited, and may be appropriately selected according to the intended purpose. Examples of the inflammation markers include C-reactive protein (CRP) and the like.

Examples of the enzymes include γ-GTP, aspartate aminotransferase (AST), alanine aminotransferase (ALT), amylase, and the like.

Examples of the hormones include adrenocorticotropic hormones, luteinizing hormones, follicle-stimulating hormones, prolactin, thyroid hormones, parathyroid hormones, aldosterone, insulin, estrogen, progesterone, growth hormones, and the like.

The inspection device of the present invention includes a hydrophobic portion and an exposed portion, and may further include other members as necessary.

### <Hydrophobic portion>

The hydrophobic portion is a portion impregnated with the hydrophobic material. The hydrophobic portion has hydrophobicity owing to the hydrophobic material with which the hydrophobic portion is impregnated, and therefore does not allow the inspection target to flow through due to capillary action.

### <Exposed portion>

The exposed portion is a portion in which the substrate for the inspection device is exposed without being impregnated with the hydrophobic material. The exposed portion preferably includes a fluid-receiving portion, a flow path portion communicating with the fluid-receiving portion, and a detection portion communicating with the flow path portion. A fluid to be applied to the fluid-receiving portion is the inspection target.

The fluid-receiving portion is a site in which the inspection target is dropped.

The detection portion is a site by which it is confirmed whether or not a target substance is present in the inspection target dropped in the fluid-receiving portion.

The fluid-receiving portion, the flow path portion, and the detection portion, which constitute the exposed portion, realize a flow of the inspection target due to capillary action.

The hydrophobic portion and the exposed portion are preferably provided on both sides of the substrate for the inspection device. Due to the above configuration, a three-dimensional flow path can be formed in the substrate.

Flow paths having mutually different designs are preferably formed in respective sides of the substrate for the inspection device, and communicate with each other to form a three-dimensional flow path.

An amount of the hydrophobic material with which the substrate for the inspection device is impregnated is preferably 15 g/m² or greater and 20 g/m² or less, and more preferably 15 g/m² or greater and 18 g/m² or less. When the amount of the hydrophobic material for the impregnation is 15 g/m² or greater and 20 g/m² or less, both formability of a flow path and flowability of a highly viscous inspection target or a suspended inspection target can be achieved. When the amount of the hydrophobic material for the impregnation is greater than 20 g/m², the hydrophobic material may permeate in a planar direction, in addition to a thickness direction, within the flow path, and therefore the hydrophobic material may block the flow path.

The impregnation of the substrate for the inspection device with the hydrophobic material is preferably performed, for example, by allowing a transfer layer of a heat transfer member to come into contact with the substrate for the inspection device and to heat-transfer the hydrophobic material included in the transfer layer.

### <Other members>

The above other members are not particularly limited, and may be appropriately selected according to the intended purpose. Examples of the other members include a protective member, and the like.

The protective member is a member for preventing contamination when the inspection device is touched by a hand. The protective member is not particularly limited, and may be appropriately selected according to the intended purpose. Examples of the protective member include a housing covering the entire inspection device, a protective film disposed on the exposed portion, such as a flow path, and the like.

A material of the protective film is not particularly limited as long as the material is easily peeled from the inspection device, and may be appropriately selected according to the intended purpose. Examples of the material of the protective film include silicone paper, polyolefin sheets (e.g., polypropylene, etc.), polytetrafluoroethylene sheets, and the like.

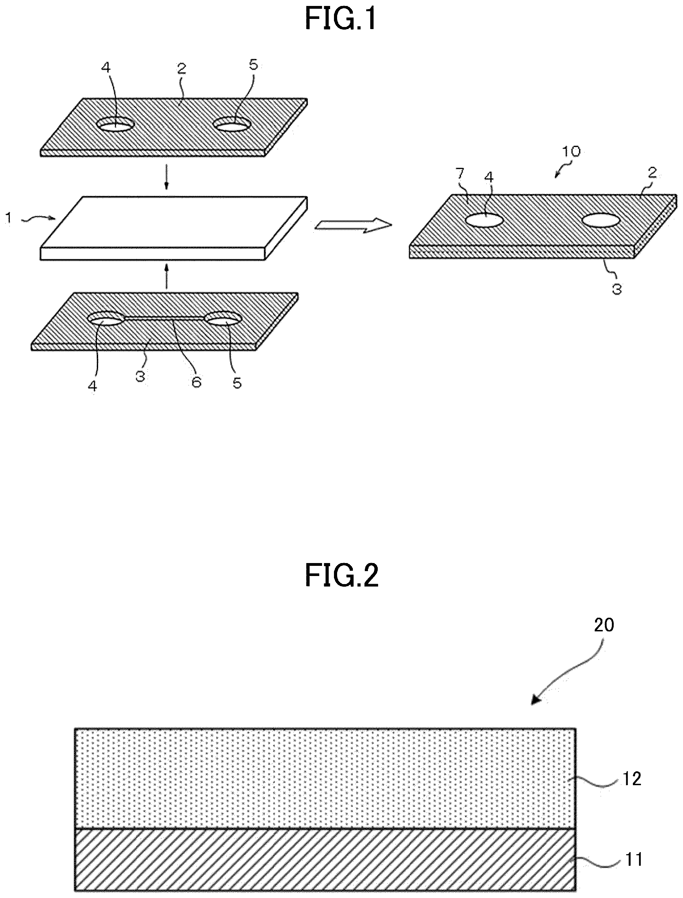

Fig. 1 is a schematic view illustrating one example of the inspection device of the present invention. Fig. 1 is a schematic exploded view of the inspection device. As illustrated in the left side of Fig. 1, the inspection device 10 includes the substrate 1 for the inspection device, a first layer 2 including the hydrophobic material, and a second layer 3 including the hydrophobic material. Moreover, the inspection device 10 has a structure in which respective sides of the substrate 1 for the inspection device are impregnated with the first layer 1 and the second layer 3.

The inspection device 10 includes a fluid-receiving portion 4, a flow path portion 6 communicating with the fluid-receiving portion, and a detection portion 5 communicating with the flow path portion, and is configured such that, when the inspection target is dropped in the fluid-receiving portion 4, the inspection target flows through the fluid-receiving portion 4, the flow path portion 6, and the detection portion 5 in this order due to capillary action.

A shape of the fluid-receiving portion 4 in a plan view is not particularly limited, and may be appropriately selected according to the intended purpose. For example, the shape of the fluid-receiving portion 4 in the plan view may be circular, elliptical, rectangular, or the like.

A shape of the detection portion 5 in a plan view is not particularly limited, and may be appropriately selected according to the intended purpose. The shape of the detection portion 5 in the plan view may be circular, elliptical, rectangular, or the like.

### (Method for manufacturing an inspection device)

The method for manufacturing the inspection device of the present invention includes an impregnation step, and may further include other steps, as necessary.

### <Impregnation step>

The impregnation step is a step of impregnating both sides of the substrate for the inspection device of the present invention with a hydrophobic material to form a hydrophobic portion impregnated with the hydrophobic material and an exposed portion in which the substrate for the inspection device is exposed without being impregnated with the hydrophobic material.

The impregnation step is performed by a thermal laminator using a heat transfer medium. For example, a heat transfer medium in which a pattern for a flow path side is cut out is fixed on one side of the substrate for the inspection device, and a heat transfer medium in which a pattern for a detection portion side is cut out is fixed on the other side of the substrate for the inspection device, and the resultant stack is passed through a thermal laminator that is set at a predetermined temperature so that the substrate is impregnated, from both sides of the substrate for the inspection device, with the hydrophobic materials included in the transfer layers of the heat transfer media in which the patterns having the different shapes are cut out to thereby form a three-dimensional flow path within the substrate.

### -Heat transfer medium-

The heat transfer medium includes a support, and a transfer layer that includes a hydrophobic material and is disposed on the support. The heat transfer medium may further include other members, as necessary.

### --Support--

A shape, structure, size, material, and the like of the support are not particularly limited, and may be appropriately selected according to the intended purpose.

Examples of the shape of the support include ribbon shapes, film shapes, and the like.

The structure of the support may be a single-layer structure, or a laminate structure.

The size of the support can be appropriately selected according to a size of the inspection device or the like.

The material of the support is not particularly limited, and may be appropriately selected according to the intended purpose. Examples of the material of the support include polyester (e.g., polyethylene terephthalate (PET), polyethylene naphthalate (PEN), etc.), polycarbonate, polyimide resins (PI), polyamide, polyethylene, polypropylene, polyvinyl chloride, polyvinylidene chloride, polystyrene, styrene-acrylonitrile copolymers, cellulose acetate, and the like. The above materials may be used alone or in combination of two or more. Among the above materials, polyethylene terephthalate (PET) and polyethylene naphthalate (PEN) are particularly preferred.

The support is not particularly limited, and may be appropriately synthesized for use, or selected from commercial products.

The average thickness of the support is not particularly limited, and may be appropriately selected according to the intended purpose. The average thickness of the support is preferably 3 µm or greater and 100 µm or less, and more preferably 5 µm or greater and 50 µm or less.

### --Transfer layer--

The transfer layer includes a hydrophobic material. The transfer layer is formed by applying the hydrophobic material to the support. The hydrophobic material is not particularly limited, as long as the hydrophobic material can be used to impregnate the substrate for the inspection device, and inhibits capillary action in the substrate for the inspection device. The hydrophobic material includes a hydrophobic substance, preferably includes a binder resin, and may further include other components, as necessary.

### ---Hydrophobic substance---

Examples of the hydrophobic substance include wax and the like.

The wax is not particularly limited, and may be appropriately selected according to the intended purpose. Examples of the wax include: natural wax, such as beeswax, carnauba wax, spermaceti, Japan wax, candelilla wax, rice bran wax, montan wax, and the like; synthetic wax, such as paraffin wax, microcrystalline wax, oxidized wax, ozokerite, ceresin, ester wax, polyethylene wax, oxidized polyethylene wax, and the like; higher fatty acids, such as margaric acid, lauric acid, myristic acid, palmitic acid, stearic acid, furoic acid, behenic acid, and the like; higher alcohols, such as stearic alcohol, behenyl alcohol, and the like; esters, such as fatty acid esters of sorbitan and the like; and amides, such as stearamide, oleamide, and the like. The above wax may be used alone or in combination of two or more.

### ---Binder resin---

The binder resin is not particularly limited, and may be appropriately selected according to the intended purpose. Examples of the binder resin include ethylene-vinyl acetate copolymers, partially saponified ethylene-vinyl acetate copolymers, ethylene-vinyl alcohol copolymers, ethylene-sodium methacrylate copolymers, polyamide, polyester, polyurethane, polyvinyl alcohol, methyl cellulose, carboxymethyl cellulose, starches, polyacrylic acid, isobutylene-maleic acid copolymers, styrene-maleic acid copolymers, polyacrylamide, polyvinyl acetal, polyvinyl chloride, polyvinylidene chloride, isoprene rubber, styrene-butadiene copolymers, ethylene-propylene copolymers, butyl rubber, acetonitrile-butadiene copolymers, and the like. The above binder resins may be used alone or in combination of two or more.

### ---Other components---

Examples of the other components include colorants, viscosity modifiers, dispersing agents, dispersion aids, fillers, and the like.

A method of forming the transfer layer is not particularly limited, and may be appropriately selected according to the intended purpose. Examples of the method include: a hot-melt coating method; a method in which a coating liquid in which the wax and the binder resin are dispersed in a solvent is applied; and the like.

When the transfer layer is formed, the hydrophobic material is preferably heated and melted.

Formation (printing) of a flow path pattern in the transfer layer on the support is not particularly limited. For example, a thermal transfer printer can be suitably used. In a first printing step, the flow path pattern is printed with a first transfer layer on a first support, where the first transfer layer is printed on the first support in a manner to have a reverse design of the first layer of the inspection device. In a second printing step, the flow path pattern is printed with a second transfer layer on a second support, where the second transfer layer is printed on the second support in a manner to have a reverse design of the second layer of the inspection device.

Note that the design to be printed is created in advance, for example, by a computer or the like, and the data can be loaded into a printing device. The printing on the front surface and the back surface of the substrate for the inspection device may be performed separately or simultaneously.

The thermal transfer printer is not particularly limited, and may be appropriately selected according to the intended purpose. Examples of the thermal transfer printer include thermal printers including serial thermal heads, thermal printers including line-type thermal heads, and the like.

### <Other steps>

The other steps are not particularly limited, and may be appropriately selected according to the intended purpose. Examples of the other steps include a drying step, a conveying step, and the like.

The inspection device of the present invention is preferably for inspecting an emulsion including droplets having a particle size of 1 µm or greater. Examples of the droplets having the particle size of 1 µm or greater include milk fat globules and the like.

Moreover, the inspection device is preferably for inspecting unhomogenized milk. Specific examples of the unhomogenized milk include cow milk that has not been subjected to homogenization.

The inspection device of the present invention using the substrate for the inspection device of the present invention can achieve both formability of a flow path, and flowability of a highly viscous inspection target or a suspended inspection target. Thus, the inspection device can inspect unhomogenized raw cow milk without causing blockage, and can detect hormone components, such as progesterone, contained in milk fat globules so that the inspection device can be suitably used as a pregnancy test kit or the like.

### Examples

Examples of the present invention will be described hereinafter, but the present invention shall not be construed as being limited to these examples.

### (Examples 1 to 4 and Comparative Examples 1 to 5)

### <Production of inspection device>

### -Preparation of hydrophobic material-

Seventy-two parts by mass of paraffin wax (Paraffin Wax-135, produced by NIPPON SEIRO CO., LTD.) serving as a hydrophobic substance, 18 parts by mass of synthetic wax ("Diacara (registered trademark) 30" produced by Mitsubishi Chemical Corporation) serving as a hydrophobic substance, 11.25 parts by mass of an ethylene-vinyl acetate copolymer resin ("Ultrathene 722", produced by Tosoh Corporation), and 1.8 parts by mass of carbon black ("MA-100", produced by Mitsubishi Chemical Corporation) serving as a colorant were blended, and the mixture was melted and mixed at 100°C. During the melting and mixing, the above ingredients were dispersed by a sand mill. In the manner as described above, a hydrophobic material was prepared.

The viscosity of the obtained hydrophobic material was measured, using a rheometer AR-G2 produced by TA Instruments Japan Inc.. under the conditions of 140°C and 3,000/s, and the viscosity was 23 mPa·s.

### -Production of heat transfer medium-

Next, a support (Lumirror 6C F531, produced by TORAY INDUSTRIES, INC., PET film, thickness: 6 µm) was placed on a hot plate whose temperature was maintained at 120°C. Subsequently, the above-described hydrophobic material which was kept in the melted state at 120°C was applied on the support by a Meyer bar (produced by MITSUI ELECTRIC CO., LTD.) so that the average thickness of the applied layer was from 5 µm to 12 µm to form a transfer layer, thereby producing a heat transfer medium 20 having the structure illustrated in Fig. **2****.** In Fig. 2, the reference numeral 11 represents the support, and the reference numeral 12 represents the transfer layer.

### -Formation (printing) of flow path-

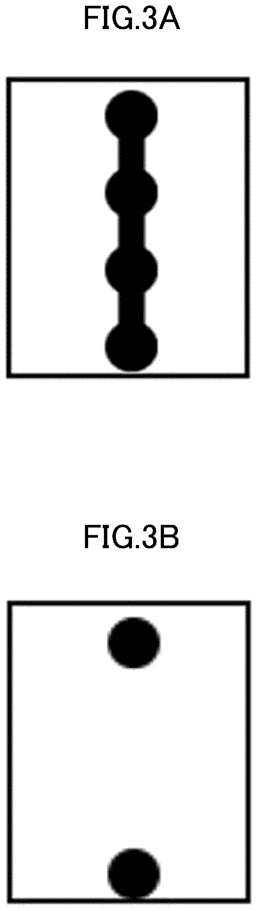

Next, a pattern illustrated in Fig. 3A, in which four circles each having a diameter of 4 mm were arranged with a gap of 6 mm between the centers of the adjacent circles, and the circles were connected with one another by a flow path having a width of 2.5 mm, was printed on the flow path (front) side of the substrate presented in Table 1 using a thermal transfer printer (Lesprit R412v-ex, produced by SATO HOLDINGS CORPORATION), and a pattern illustrated in Fig. 3B, in which the uppermost and the lowermost circles of the flow path were passed through with circles of the same diameter, respectively, was printed on the detection portion (back) side of the substrate presented in Table 1 using the above thermal transfer printer, to thereby form a flow path pattern in which the hydrophobic material of the printed part was cut out from the heat transfer medium.

### -Formation of flow path in substrate-

The heat transfer medium from which the pattern of the flow path side was cut out was fixed to one side of the substrate, and the heat transfer medium from which the pattern of the detection portion side was cut out was fixed to the opposite side of the substrate, and the resultant stack was passed through a thermal laminator so that the substrate was impregnated, from the front and back, with the hydrophobic material of the heat transfer media which were cut out in the different shapes to thereby form a three-dimensional flow path within the substrate.

### -Thermal laminate conditions-

Thermal lamination was performed using GL535ML produced by GBC as a thermal laminator.
(1) Thermal lamination was performed at a line speed of 10 mm/sec with a transfer temperature of the hydrophobic material to the substrate being 85°C or higher and 120°C or lower.
(2) Thermal lamination was performed at a line speed of 5 mm/sec with a transfer temperature of the hydrophobic material to the substrate being 85°C or higher and 120°C or lower.

### -Formation of flow path with different amount of hydrophobic material in substrate-

As presented in Table 1, each flow path was formed by adjusting an amount of the hydrophobic material used for impregnation, by using a combination of a weight (thickness) of the hydrophobic material of the heat transfer medium used for forming the pattern of the flow path side, and a weight (thickness) of the hydrophobic material of the heat transfer medium used for forming the pattern of the detection portion side.

In the manner as described above, inspection devices of Examples 1 to 4 and Comparative Examples 1 to 5 were produced.

Next, each of the produced inspection devices was subjected to evaluations of various properties in the following manner. The results are presented in Table 2.

### <Barrier properties>

In each of the produced inspection devices, 16 µL of a solution in which an aqueous fluorescent ink extracted from a fluorescent marker pen produced by ASKUL Corporation was dissolved in distilled water (inspection target) was dropped into the fluid-receiving portion with a dropper and was passed through the flow path. The presence or absence of leakage of the solution from the flow path was visually observed using a UV lamp (BL-LED3435-UV, produced by CONTEC CO., LTD.), and determined according to the following criteria.

### [Evaluation criteria]

A: There was no leakage from the flow path, and the barrier properties were desirable.
B: Leakage from the flow path occurred slightly, but at a level that did not cause any problem in practice.
C: Leakage from the flow path occurred and barrier properties were not desirable.

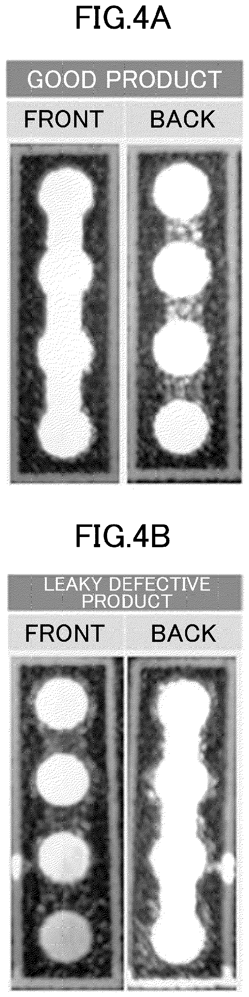

In the case where the filter paper is not sufficiently impregnated with the hydrophobic material, the inspection target leaks out from the flow path. Thus, the degree of leakage of the inspection target out from the flow path was observed. Fig. 4A illustrates a state of Example 1 in which barrier properties were desirable, as leakage of the inspection target from the flow path did not occur. Fig. 4B illustrates a state of Comparative Example 2 in which barrier properties were not desirable, as there was leakage of the inspection target from the flow path.

### <Fluid flowability>

In each of the inspection devices, 16 µL of a solution in which unhomogenized raw cow milk and the above aqueous fluorescent ink were dissolved in distilled water was dropped in the fluid-receiving portion with a dropper, and the time until the dripped solution reached the detection portion was measured with a stopwatch. The case where the solution did not reach the detection portion within 3 minutes (180 seconds) was determined as "blockage." The number of evaluations was n = 3, the flow time was the average value among n = 3, and when "blockage" was confirmed in at least one of n = 3, the result was determined as "blockage."

### <Paper strength>

Whether or not each of the produced inspection devices could be cut into a predetermined shape using a cutter (Hyper AL type, produced by Olfa Corporation) was evaluated based on the following criteria.

### [Evaluation criteria]

A: The inspection device could be easily cut with the cutter.
AB: The inspection device could be cut with the cutter.
B: It was difficult to cut the inspection device with the cutter.
C: The inspection device could not be cut with the cutter (the fibers had weak cohesive force and broke)

**[Table 1]**

| | Type of substrate | Average thickness [µm] | Basis weight [g/m²] | Density [ × 10⁶(g/m³)] | Amount of hydrophobic material for impregnation (flow path side/ detection side) [g/m²] |
|---|---|---|---|---|---|
| Ex. 1 | Sample 1 | 250 | 79 | 0.31 | 17.6(8.8/8.8) |
| Ex. 2 | Sample 2 | 290 | 93 | 0.32 | 17.6(8.8/8.8) |
| Ex. 3 | Sample 3 | 300 | 88 | 0.29 | 17.6(8.8/8.8) |
| Ex. 4 | Sample 3 | 300 | 88 | 0.29 | 13.6(8.8/4.8) |
| Comp. Ex. 1 | Whatman #41 | 220 | 85 | 0.39 | 17.6(8.8/8.8) |
| Comp. Ex. 2 | Advantec No.60 | 560 | 125 | 0.22 | 17.6(8.8/8.8) |
| Comp. Ex. 3 | Sample 4 | 210 | 49 | 0.23 | 17.6(8.8/8.8) |
| Comp. Ex. 4 | Whatman #40 | 210 | 95 | 0.45 | 17.6(8.8/8.8) |
| Comp. Ex. 5 | Whatman #42 | 200 | 100 | 0.50 | 17.6(8.8/8.8) |

**[Table 2]**

| | Fluid flowability (s) | | Barrier properties | Paper strength |
|---|---|---|---|---|
| | Water | Unhomogenized milk | | |
| Ex. 1 | 8.7 | 38.3 | A | B |
| Ex. 2 | 14.7 | 42.7 | A | A |
| Ex. 3 | 16.9 | 51.1 | A | A |
| Ex. 4 | Slightly leaked | 34.3 | B | A |
| Comp. Ex. 1 | 33.5 | Blocked | A | A |
| Comp. Ex. 2 | Leaked | 31.4 | C | A |
| Comp. Ex. 3 | Blocked | Blocked | A | C |
| Comp. Ex. 4 | 100.5 | Blocked | A | A |
| Comp. Ex. 5 | Blocked | Blocked | A | A |

### -Substrate-

Samples 1 to 4: Filter paper prepared to have the average thickness, basis weight, and density presented in Table 1.
Whatman #40, #41, #42: Commercially available quantitative filter paper, produced by Merck
Advantec No. 60: Commercially available filter paper for viscous fluids, produced by ADVANTEC TOYO KAISHA, LTD.

It was found from the results of Tables 1 and 2 that, in Examples 1 to 3, the average thickness of the substrate was 250 µm or greater and 350 µm or less, the density was 0.25 × 10⁶ g/m³ or greater and 0.40 × 10⁶ g/m³ or less, and desirable fluid flowability, barrier properties, and paper strength were achieved.

It was found that, in Example 4, the amount of the hydrophobic material for impregnation did not satisfy being 15 g/m² or greater and 20 g/m² or less, and therefore the barrier properties and flowability of water were worse than in Examples 1 to 3.

In Comparative Example 1, conversely, the flow path was blocked with the unhomogenized raw cow milk, since the average thickness of the substrate was thin.

In Comparative Example 2, it was found that, since the average thickness of the substrate was thick and the density was low, the barrier properties and flowability of water were degraded.

In Comparative Example 3, it was found that, since the average thickness of the substrate was thin and the density was low, the flow path was blocked with water and the unhomogenized raw cow milk, and the paper strength became weak.

In Comparative Examples 4 and 5, since the average thickness of the substrate was thin and the density was high, the flow time was significantly prolonged, and the flow path was blocked with the unhomogenized raw cow milk.

### INDUSTRIAL APPLICABILITY

Since the inspection device of the present invention using the substrate for the inspection device of the present invention can achieve both flow path formability and flowability of a highly viscous inspection target and a suspended inspection target, unhomogenized raw cow milk can be inspected without causing a blockage, and hormone components, such as progesterone, included in milk fat globules can be detected so that the inspection device can be applied to pregnancy test kits or the like.

The present application claims priority based on Japanese Patent Application No. 2022-100746, filed on June 23, 2022, the entire content of which are incorporated herein by reference.

### REFERENCE SIGNS LIST

- 1: substrate for inspection device
- 2: first layer
- 3: second layer
- 4: fluid-receiving portion
- 5: detection portion
- 6: flow path portion
- 10: inspection device
- 11: support
- 12: transfer layer
- 20: heat transfer medium

## Claims

1. A substrate for an inspection device, the substrate having:
an average thickness of 250 µm or greater and 350 µm or less; and
a density of 0.25 × 10⁶ g/m³ or greater and 0.40 × 10⁶ g/m³ or less.

2. The substrate for the inspection device according to claim 1,
wherein the substrate has a basis weight of 50 g/m² or greater and 100 g/m² or less.

3. The substrate for the inspection device according to claim 1 or 2,
wherein the substrate includes at least one selected from a group consisting of filter paper, woodfree paper, a nonwoven fabric, and nitrocellulose.

4. An inspection device for inspecting a presence or absence of a target substance in an inspection target, the inspection device comprising:
a hydrophobic material; and
the substrate for the inspection device of claim 1 or 2, impregnated with the hydrophobic material,
wherein the inspection device includes a hydrophobic portion impregnated with the hydrophobic material, and an exposed portion in which the substrate is exposed without being impregnated with the hydrophobic material.

5. The inspection device according to claim 4,
wherein the exposed portion includes a fluid-receiving portion, a flow path portion communicating with the fluid-receiving portion, and a detection portion communicating with the flow path portion.

6. The inspection device according to claim 4,
wherein the hydrophobic portion and the exposed portion are provided to both sides of the substrate for the inspection device, and
flow paths having mutually different designs are formed in respective sides of the substrate for the inspection device, and the flow paths communicate with each other to form a three-dimensional flow path.

7. The inspection device according to claim 4,
wherein an amount of the hydrophobic material with which the substrate for the inspection device is impregnated is 15 g/m² or greater and 20 g/m² or less.

8. The inspection device according to claim 4,
wherein the inspection device is for inspecting an emulsion including droplets having a particle size of 1 µm or greater.

9. The inspection device according to claim 4,
wherein the inspection device is for inspecting unhomogenized milk.

10. A method for manufacturing an inspection device, the method comprising:
impregnating both sides of the substrate for the inspection device of claim 1 or 2 with a hydrophobic material to form a hydrophobic portion impregnated with the hydrophobic material and an exposed portion in which the substrate for the inspection device is exposed without being impregnated with the hydrophobic material.
